# EUROPEAN PATENT APPLICATION

(11) **EP 4 768 000 A1**
(43) Date of publication of application: **01.07.2026**
(21) Application number: 25162639.6
(22) Date of filing: 10.03.2025
(51) Int. Cl.: A61F 9/007

(54) **IMPLANT DEVICE FOR EYE DISEASE WITH OPTIMIZED INNER DIAMETER OF TUBE AND DIAMETER OF RIPCORD AND METHOD FOR MAINTAINING INTRAOCULAR PRESSURE OF SUBJECT USING THE SAME**

(30) Priority: 26.12.2024 KR 20240196522
(71) Applicant: Microt Inc., Seoul 06351 (KR)
(72) Inventor: LEE, Eun Suk, Seongnam (KR); LEE, Ye Ji, Pyeongchang (KR)
(74) Representative: Botti & Ferrari S.p.A.

(57) **Abstract**

An implant device (10) for an eye disease to be inserted into an eyeball may include: a tube (11) having one end to be inserted into an anterior chamber of the eyeball, the tube comprising a hollow portion through which aqueous humor is drained; and a ripcord (13) inserted into the hollow portion of the tube to control an amount of aqueous humor drainage through the tube. In the implant device, an inner diameter of the tube and a diameter of the ripcord are determined so that pressure formed in the anterior chamber of the eyeball when the tube is inserted into the anterior chamber of the eyeball is within a preset pressure range. The pressure formed in an anterior chamber to an optimal range through an appropriate combination of the tube and the ripcord inserted into the tube.

## Description

### CROSS-REFERENCE TO RELATED APPLICATION

This application claims priority to Korean Patent Application No. 10-2024-0196522, filed on December 26, 2024, and all the benefits accruing therefrom under 35 U.S.C. §119, the contents of which in its entirety are herein incorporated by reference.

### BACKGROUND

### 1. Field

The present disclosure relates to an implant device for an eye disease and a method for maintaining intraocular pressure of a patient. More particularly, the present disclosure relates to an implant device for an eye disease configured to control pressure formed in an anterior chamber to an optimal range through an appropriate combination of a tube and a ripcord inserted into the tube in a method of lowering intraocular pressure by draining aqueous humor through the tube inserted into an eyeball, and technology for maintaining intraocular pressure of a patient at a preset intraocular pressure by using the implant device.

### 2. Description of the Related Art

For a glaucoma patient whose intraocular pressure is not controlled even by using an intraocular pressure lowering agent, intraocular pressure is lowered by creating a bypass to drain aqueous humor from an anterior chamber of an eye to an external surface of the eye under the conjunctiva. Trabeculectomy among glaucoma filtration surgeries that create a bypass or a fistula for aqueous humor drainage may fail to control intraocular pressure when the amount of aqueous humor drainage decreases due to closure of the bypass after surgery. When initial surgery fails and glaucoma filtration surgery is performed again, the frequency of bypass closure after surgery increases and a success rate of surgery is low.

Also, even in the case of intractable glaucoma such as neovascular glaucoma or secondary glaucoma caused by uveitis according to types of glaucoma, closure of a bypass frequently occurs after trabeculectomy, resulting in poor results. For an eye with a history of failed glaucoma filtration surgery or intractable glaucoma, glaucoma implant surgery of locating a glaucoma implant device is performed to prevent closure of a bypass and increase a success rate of surgery. To date, glaucoma implant surgery has been used as an alternative to trabeculectomy, especially in several difficult-to-treat glaucoma, in that glaucoma implant surgery not only effectively lowers intraocular pressure but also shows a predictable postoperative clinical course according to an inner diameter of a given tube.

However, an existing glaucoma implant used for glaucoma implant surgery may cause various problems and complications such as difficulty in surgery due to a relatively large size, postoperative exposure, infection, eye movement disorder due to a large body, and diplopia. Accordingly, small-sized glaucoma implant instruments for minimally-invasive glaucoma surgery (MIGS) have recently been developed to relatively easily lower intraocular pressure by using a glaucoma implant and to reduce side effects after surgery due to a large size.

MIGS is a method of inserting a tube with an inner diameter of micrometers into an anterior chamber of an eye to drain aqueous humor from the anterior chamber. When a proximal end of the tube exposed to the outside of an eyeball directly contacts a sclera and Tenon's capsule, a wound may open and burst. Also, there is a possibility that the tube may become blocked due to fibrosis of tissue surrounding the eye at an end of the tube inserted into the eyeball, a surface where the tube is sutured, and an aqueous humor outlet of the exposed tube. When the aqueous humor outlet of the tube is blocked in this way, the aqueous humor may not be drained through the MIGS tube, causing a glaucoma patient's intraocular pressure to rise again. To solve this problem, reoperation is required to open a fibrotic area or re-insert an implant. In particular, when the tube is sutured to the eyeball, there is a high risk that the outlet will gradually become blocked even if the aqueous humor initially flows out through the tube.

Also, in the MIGS, a ripcord may be inserted into the tube to control the amount of aqueous humor drainage. The ripcord is used to prevent initial hypotony after implant surgery and should be removed after a certain period of time (e.g., about 4 weeks to about 16 weeks).

However, in order for the aqueous humor to be drained from the eyeball at an appropriate pressure, the diameter, length, etc. of each portion of the glaucoma implant having a very small size should be carefully adjusted, and when the size is not carefully designed, the aqueous humor may be excessively drained, causing hypotony, or the aqueous humor may not be sufficiently drained. However, sufficient research has not yet been conducted on a design of a glaucoma implant instrument optimized for formed anterior chamber pressure.

### SUMMARY

In one aspect of the present disclosure, an implant device for an eye disease is provided. The implant device is configured to control pressure formed in an anterior chamber to an appropriate range through an appropriate combination of a tube and a ripcord inserted into the tube in a method of lowering intraocular pressure by draining aqueous humor through the tube inserted into an eyeball.

Also, in one aspect of the present disclosure, the implant device for an eye disease in which an inner diameter of a tube and a diameter of a ripcord inserted into the tube are combined to have an optimal numerical range is provided.

According to an embodiment, an implant device for an eye disease to be inserted into an eyeball may comprise: a tube having one end to be inserted into an anterior chamber of the eyeball, the tube comprising a hollow portion through which aqueous humor is drained; and a ripcord to be inserted into the hollow portion of the tube to control an amount of aqueous humor drainage through the tube.

Herein, an inner diameter of the tube and a diameter of the ripcord are determined so that pressure formed in the anterior chamber of the eyeball when the tube is inserted into the anterior chamber of the eyeball is within a preset pressure range.

According to an embodiment, the preset pressure range is about 6 mmHg to about 20 mmHg.

According to an embodiment, a length of the tube is about 4 mm to about 10 mm, the inner diameter of the tube is about 60 µm to about 220 µm, and the diameter of the ripcord is about 1 µm to about 180 µm.

According to an embodiment, a length of the tube is about 5 mm to about 8 mm, the inner diameter of the tube is about 60 µm to about 140 µm, and the diameter of the ripcord is about 1 µm to about 130 µm.

According to an embodiment, at least one of the inner diameter of the tube and the diameter of the ripcord is determined so that a ratio of the diameter of the ripcord to the inner diameter of the tube is between a preset lower limit and a preset upper limit.

According to an embodiment, the ratio of the diameter of the ripcord to the inner diameter of the tube is about 1:0.01 to about 1:0.9. According to an embodiment, at least one of the preset upper limit and the preset lower limit is determined to increase as the inner diameter of the tube increases.

According to an embodiment, an increment in the at least one of the preset upper limit and the preset lower limit, as the inner diameter of the tube increases, decreases as the inner diameter of the tube increases.

According to an embodiment, at least one of the preset upper limit and the preset lower limit is determined to decrease as a length of the tube increases.

According to an embodiment, at least one of the inner diameter of the tube and the diameter of the ripcord is determined so that a ratio of a square of the diameter of the ripcord to a square of the inner diameter of the tube is between a preset lower limit and a preset upper limit.

According to an embodiment, the implant device for an eye disease further comprises a wing coupled to the tube to be located at a preset distance from a distal end of the tube and extending in a direction different from a longitudinal direction of the tube.

According to an aspect of the present disclosure, a method for maintaining a predetermined intraocular pressure of a subject in need thereof comprises: inserting an implant device for an eye disease according to any of the preceding claims into an anterior chamber of an eyeball of the subject.

An implant device for an eye disease according to one aspect of the present disclosure may solve problems such as low intraocular pressure due to excessive drainage of aqueous humor or insufficient drainage of aqueous humor by combining an inner diameter of a tube and a diameter of a ripcord inserted into the tube to achieve optimal anterior chamber pressure, in lowering intraocular pressure by draining aqueous humor into conjunctival tissue or Tenon's tissue through the tube inserted into an anterior chamber of an eye.

### BRIEF DESCRIPTION OF THE DRAWINGS

FIGS. 1 and 2 are conceptual views illustrating a state where an implant device for an eye disease is inserted into an eyeball according to an embodiment.
FIG. 3A is a perspective view illustrating an implant device for an eye disease according to an embodiment.
FIG. 3B is a cross-sectional view illustrating a wing portion of the implant device for an eye disease of FIG. 3A.
FIG. 4 is a plan view for describing shapes of an implant device for an eye disease according to embodiments.

### DETAILED DESCRIPTION

The terms used herein will be briefly described, and the present disclosure will be described in detail.

The terms used herein are general terms currently widely used in the art in consideration of functions in the present disclosure, but the terms may vary according to the intention of one of ordinary skill in the art, precedents, or new technology in the art. Also, some of the terms used herein may be arbitrarily chosen by the present applicant, and in this case, these terms are defined in detail below. Accordingly, the specific terms used herein should be defined based on the unique meanings thereof and the whole context of the present disclosure.

It will be understood that when a certain part "includes" a certain component, the part does not exclude another component but may further include another component, unless the context clearly dictates otherwise. Also, throughout the specification, when an element is referred to as being "connected" to another element, it will be understood to include that the element is "directly connected" to the other element or is "connected" to the other element with another element therebetween.

The present disclosure will now be described more fully with reference to the accompanying drawings for one of ordinary skill in the art to be able to perform the present disclosure without any difficulty. However, the present disclosure may be embodied in many different forms and is not limited to the embodiments set forth herein. For clarity, portions irrelevant to the descriptions of the present disclosure are omitted in the drawings, and like components are denoted by like reference numerals throughout the specification.

Hereinafter, the present disclosure will be described in detail with reference to the accompanying drawings.

### Implant for eye disease for minimally-invasive glaucoma surgery (MIGS)

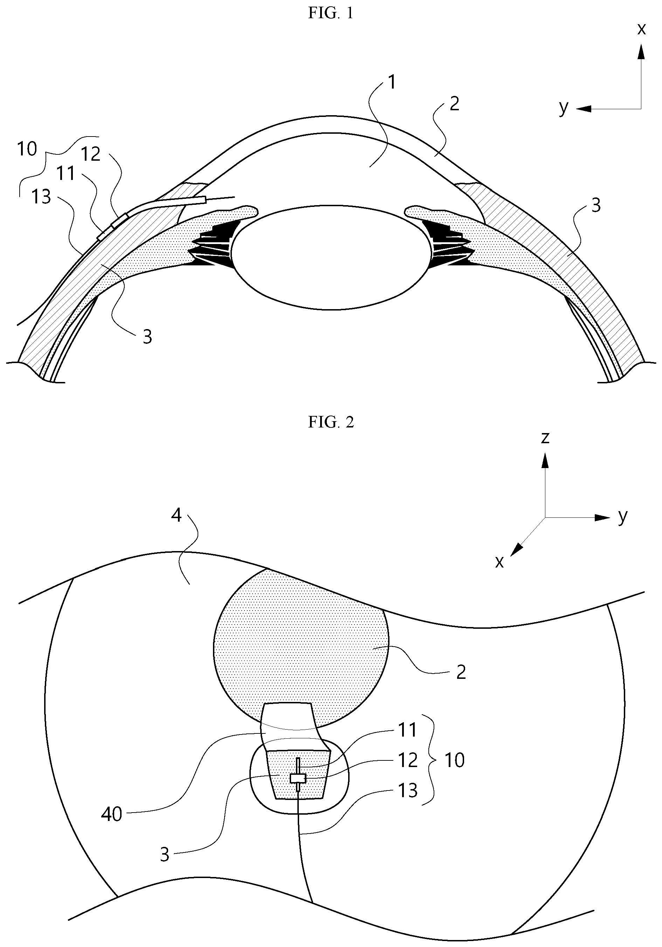

FIGS. 1 and 2 are conceptual views for describing an implant device used in a surgical method for inserting an implant for an eye disease and a state where the implant device is inserted into an eye according to an embodiment.

Referring to FIGS. 1 and 2, an implant device 10 for an eye disease is to be at least partially inserted into a sclera 3 of an eyeball. In this specification, the implant device 10 used in a surgical method according to embodiments will be described by using an implant for glaucoma treatment as an example. However, the implant device 10 for an eye disease used in the surgical method according to embodiments of the present disclosure may be used to treat or alleviate symptoms of various eye diseases that cause or caused by increased intraocular pressure.

When used to treat glaucoma, the implant device 10 is intended to control intraocular pressure by controlling the amount of aqueous humor drainage in an anterior chamber 1 located in front of a lens and below a cornea 2 in an eyeball, thereby preventing damage to an optic nerve due to increased intraocular pressure caused by eye diseases.

Eye diseases in the specification may include glaucoma caused by an increase in intraocular pressure. Examples of glaucoma may include, but are not limited to, congenital glaucoma, traumatic glaucoma, glaucoma suspect, ocular hypertension, primary open-angle glaucoma, normal-tension glaucoma, capsular glaucoma with pseudoexfoliation of lens, chronic simple glaucoma, low-tension glaucoma, pigmentary glaucoma, primary angle-closure glaucoma, acute angle-closure glaucoma, chronic angle-closure glaucoma, intermittent angle-closure glaucoma, glaucoma secondary to eye trauma, glaucoma secondary to eye inflammation, glaucoma secondary to drugs, neovascular glaucoma, and secondary glaucoma due to uveitis.

In an embodiment, the implant device 10 may include a tube 11 applicable to minimally-invasive glaucoma surgery (MIGS), and one end of the tube 11 may be inserted into the anterior chamber 1 of the eyeball and the other end of the tube 11 may be located on conjunctival tissue or Tenon's tissue. The tube 11 may include a hollow portion through which aqueous humor of the eyeball may flow, and may allow the aqueous humor to be drained from the anterior chamber 1 of the eyeball to the outside through the tube 11.

In more detail, the tube 11 may be inserted into the eyeball so that among both ends of the tube 11, a distal end is located in the anterior chamber 1 of the eyeball and a proximal end is located on the conjunctival tissue or the Tenon's tissue of the eyeball to drain the aqueous humor generated in the anterior chamber of the eyeball to the conjunctival tissue or the Tenon's tissue through the hollow portion of the tube 11. In the specification, the distal end and proximal end are defined according to a direction from an operator inserting the implant device 10, and among both ends of the tube 11, the proximal end refers to an end facing the operator and the distal end refers to an end facing a patient's eye into which the implant device 10 is to be inserted.

In an embodiment, the tube 11 may be formed of a biocompatible material and may be formed of a changeable material. Also, because the tube 11 may affect the flow of the aqueous humor through the hollow portion of the tube 11 when the tube 11 is formed of a viscous material, the tube 11 may be formed of a material with a smooth surface that does not affect the flow of the aqueous humor.

For example, the tube 11 may be formed of a silicone or other silicon-based material, a urethane-based material such as PTFE, polycarbonate, or polyurethane (PU), a composite material of a silicone-based material and a polyurethane-based material such as silicone-PU, or a biocompatible metal or alloy.

Also, in an embodiment, the tube 11 may be formed of, but is not limited to, silicone, PTFE, polycarbonate, polyurethane, polyethylene, polypropylene, polyimide, PMMA, poly(styrene-b-isobutylene-b-styrene), polyethersulfone, gelatin, stainless steel, titanium, titanium, nitinol, or a combination thereof.

In an embodiment, the tube 11 may be formed in a curved shape with a certain curvature in order to prevent damage to the endothelium of the cornea in the eyeball. A front end of the tube may poke and damage the cornea in the anterior chamber of the eyeball in a process of inserting the tube into the anterior chamber of the eyeball according to a size of the eyeball that is different for each patient, a skill level in tube injection, etc. Damage to the cornea may cause complications such as corneal decompensation which require even future corneal transplantation. According to the present embodiment, in order for the tube to naturally move in a curved shape while being inserted into the anterior chamber of the eyeball, the tube 11 may be manufactured in a curved shape with a certain curvature corresponding to a curvature of a surface of the eyeball.

A ripcord 13 is inserted into the tube 11. The ripcord 13 is an intraocular pressure control member inserted into the hollow portion of the tube 11 to control the flow of the aqueous humor through the tube 11, and maintains intraocular pressure of the patient into which the implant device 10 for an eye disease is inserted at a preset intraocular pressure. In an embodiment, the ripcord 13 may have a cylindrical shape with a circular cross-section, but a cross-sectional shape of the ripcord 13 is not limited thereto.

In an embodiment, the implant device 10 includes a wing 12 coupled to an outer surface of the tube 11 and having a cross-section that is at least partially larger than a diameter of the tube 11. As a result, a part of the wing 12 protrudes in a lateral direction of the tube 11, that is, in a direction different from a longitudinal direction of the tube 11. Due to the presence of the wing 12 extending in the lateral direction of the tube 11, when the tube 11 is pushed toward the sclera 3 of the eyeball, the wing 12 may be caught on the sclera 3 and may prevent the tube 11 from being completely inserted into the eyeball. The wing 12 may be formed by coupling or adhering one or more members to the surface of the tube 11, or may be formed by inserting the tube 11 into a member extending in a direction different from the longitudinal direction of the tube 11.

Referring to FIG. 2, when the surgical method according to embodiments is used, the implant device 10 for an eye disease may be inserted into the sclera 3 through an area under a scleral flap 40 formed after an operator exfoliates a part of a limbus 4 surrounding the cornea 2 of the eyeball. After the implant device 10 is inserted, the implant device 10 may be disposed in the eyeball by covering the scleral flap 40 again.

That is, after the scleral flap 40 is created by cutting the limbus 4 of the sclera 3 surrounding the cornea 2, the created scleral flap 40 may be lifted and the distal end of the tube 11 may be inserted to pass through the exposed sclera 3. In this case, the tube 11 is not completely inserted into the sclera 3, and the proximal end of the tube 11 including an aqueous humor outlet is located outside the sclera 3. After one end of the tube 11 is inserted into the anterior chamber 1 through the sclera 3, the lifted scleral flap 40 may be lowered. When the implant device 10 for an eye disease is inserted into the eyeball, the aqueous humor generated in the anterior chamber may flow through the tube 11 of the implant device for an eye disease, thereby draining the aqueous humor from the anterior chamber and lowering intraocular pressure.

Hereinafter, in the specification, when the implant device 10 or the tube 11 of the implant device 10 is inserted into the eyeball, it means that at least a part of the implant device 10 or the tube 11 of the implant device 10 is located in the sclera 3 of the eyeball. In contrast, when at least a part of the implant device 10 or the tube 11 of the implant device 10 is located outside the eyeball, it means that the part is located outside the sclera 3 of the eyeball. However, when it is described that a part is located outside the eyeball, it includes a case where the part is covered by the scleral flap 40 outside the sclera 3.

Also, the implant device 10 for an eye disease further includes the ripcord 13 inserted into the hollow portion of the tube 11. The ripcord 13 is for controlling pressure formed in the anterior chamber through the hollow portion of the tube 11. In an embodiment, the ripcord 13 may be a non-absorbable surgical suture thread, and may be formed of a nylon or prolene material, but the present disclosure is not limited thereto.

When the ripcord 13 is inserted into the hollow portion of the tube 11, the flow of the aqueous humor becomes unsmooth, causing the aqueous humor to be accumulated in the anterior chamber of the eye, and intraocular pressure is relatively increased compared to when the ripcord 13 is not present. In the specification, pressure formed in the anterior chamber refers to pressure in the anterior chamber of the eyeball formed at this time. The implant device10 for an eye disease may be inserted into the eyeball with the ripcord 13 inserted into the tube 11, or the implant device 10 for an eye disease, excluding the ripcord 13, may be inserted into the eye and then the ripcord 13 may be inserted into the tube 11.

In an embodiment, an insertion position of the implant device 10 may be determined so that the proximal end of the tube 11 of the implant device 10 exposed outside the sclera 3 is not completely covered by the scleral flap 40. Also, in an embodiment, a part of the scleral flap 40 covering the tube 11 exposed outside the sclera 3 may be lifted upward (i.e., toward the outside of the eyeball) through a process of suturing a part of the scleral flap 40 to another part of the scleral flap 40, the eyeball, or the implant device 10.

### Configuration of implant device for eye disease

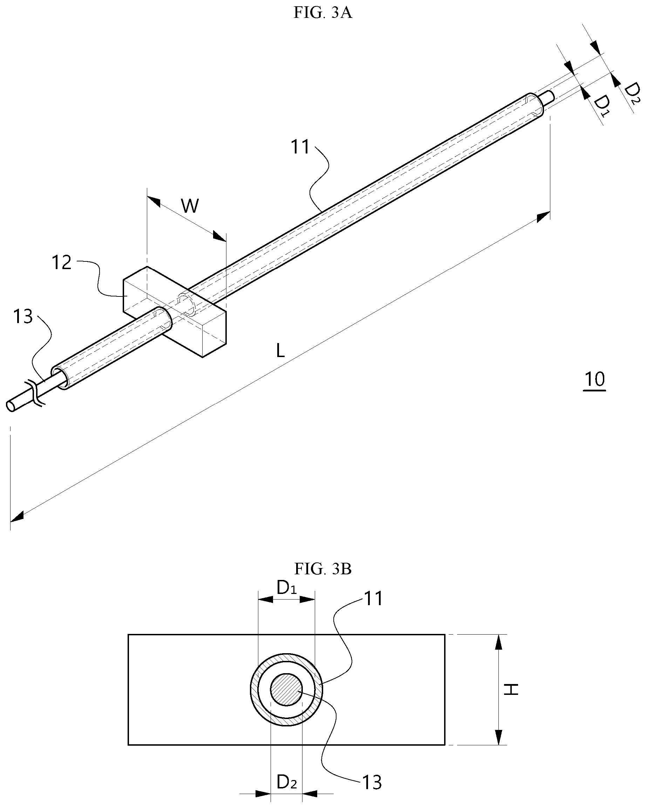

FIG. 3A is a perspective view illustrating an implant device for an eye disease according to an embodiment. FIG. 3B is a cross-sectional view illustrating a wing portion of the implant device for an eye disease of FIG. 3.

Referring to FIGS. 3A and 3B, the implant device 10 for an eye disease according to the present embodiment includes the tube 11 applicable to MIGS and the ripcord 13 inserted into the tube 11. In an embodiment, the implant device 10 for an eye disease further includes the wing 12 coupled to the tube 11.

The tube 11 is inserted into an eyeball so that a distal end (a right end in FIG. 3A) is located in an anterior chamber of the eyeball and a proximal end (a left end in FIG. 3A) is located on conjunctival tissue or Tenon's tissue of the eyeball, and the tube 11 allows aqueous humor generated in the anterior chamber of the eyeball to be drained through a hollow portion in the tube 11 to the conjunctival tissue or the Tennon's tissue. In the specification, the distal end and the proximal end are defined according to a direction from an operator inserting the implant device, and among both ends of the tube 11, the proximal end refers to an end facing the operator and the distal end refers to an end facing a patient's eye into which the implant device 10 is to be inserted.

The wing 12 is coupled to an outer surface of the tube 11, and at least a portion of the wing 12 has a cross-section larger than a diameter of the tube 11. As a result, a part of the wing 12 protrudes in a lateral direction of the tube 11, that is, in a direction different from a longitudinal direction of the tube 11. A size of the wing 12 may be appropriately determined so that the wing 12 extends in the lateral direction of the tube 11 and when the tube 11 is pushed toward a sclera of the eyeball, the wing 12 is caught on the sclera to prevent movement of the tube 11.

The wing 12 may be permanently coupled to the tube 11 or may be detachably coupled to the tube 11. In an embodiment, the wing 12 may be coupled to the surface of the tube 11 by using an adhesive material.

In another embodiment, the wing 12 is a block-shaped member having a hole through which the tube 11 passes, and the tube 11 may be coupled to the wing 12 by inserting the tube 11 into the hole formed in the block-shaped member. In this case, both ends of the wing 12 may protrude in the lateral direction of the tube 11 so that the wing 12 is caught on the sclera and the tube 11 is prevented from being completely inserted into the eyeball during a MIGS procedure. Also, a thickness of the wing 12 may be determined to be small enough (e.g., about 350 µm) to prevent the patient from feeling a foreign matter even when the wing 12 is inserted into the conjunctival tissue or the Tenon's tissue.

In still another embodiment, the tube 11 and the wing 12 may be integrally manufactured from the beginning by using a method such as extrusion or injection using synthetic resin.

The ripcord 13 is at least partially inserted into the hollow portion of the tube 11 to control the amount of aqueous humor drainage through the tube 11. When the ripcord 13 is thick, a space between an inner wall of the tube 11 and the ripcord 13 becomes narrow and the aqueous humor is relatively slowly drained, and thus pressure formed in the anterior chamber increases. When the ripcord 13 is thin, a space between the inner wall of the tube 11 and the ripcord 13 becomes wide and the aqueous humor is relatively rapidly drained, and thus, pressure formed in the anterior chamber decreases. Accordingly, pressure formed in the anterior chamber may be optimized to be within a certain range, for example, postoperative pressure is about 6 mmHg to about 20 mmHg or any number or subrange therebetween through an appropriate configuration of the ripcord 13, and in the specification, this pressure formed in the anterior chamber is referred to as normal intraocular pressure.

However, a preferred numerical range of pressure formed in the anterior chamber formed by the implant device for an eye disease according to embodiments is not limited thereto.

Embodiments of the present disclosure aim to provide a design of the implant device 10 that may maintain pressure of aqueous humor drainage through the tube 11 (i.e., pressure formed in the anterior chamber) within a certain range, by optimizing a numerical range of each of an inner diameter D₁ of the tube 11 and a diameter D₂ of the ripcord 13, which will be described below in detail.

Also, the ripcord 13 may be manipulated by a clinician to control pressure formed in the anterior chamber. For example, when the ripcord 13 is inserted into the hollow portion of the tube 11 and exposed from a rear end of the tube 11, the clinician may control the amount of aqueous humor drainage by adjusting the ripcord 13 exposed from the rear end of the tube 11. That is, the clinician may appropriately control intraocular pressure according to the patient's condition by using the ripcord 13.

In an embodiment, the ripcord 13 may be formed of a material whose shape (e.g., the diameter D₂) may be maintained without deformation even when the ripcord 13 contacts the aqueous humor. Also, the ripcord 13 may be formed of a relatively rigid material so that the ripcord 13 does not stick to an inner surface of the tube 11 when the clinician removes the ripcord 13 from the tube 11. For example, when the tube 11 is formed of an elastomer, the ripcord 13 may be formed of a relatively hard material compared to the elastomer.

In an embodiment, in order to prevent a user from feeling a foreign matter due to the ripcord 13 exposed to the outside of the tube 11, the ripcord 13 may be formed so that a diameter gradually decreases from a point where the ripcord 13 is exposed from the tube 11 or an implant body (not shown) coupled to the rear end of the tube 11.

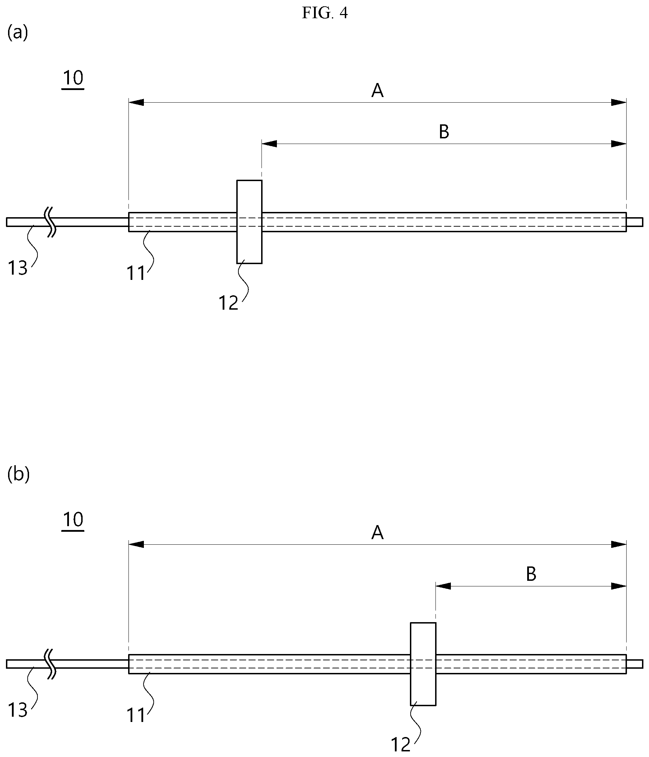

FIG. 4 is a plan view for describing various shapes of an implant device for an eye disease according to embodiments.

Referring to FIGS. 3 and 4, in an embodiment, a length A of the tube 11 suitable for use in the implant device 10 for an eye disease is about 4 mm to about 10 mm or any number or subrange therebetween. When the length A of the tube 11 is too long, aqueous humor may not be smoothly drained, and in contrast, when the length A of the tube 11 is too short, the aqueous humor may be excessively drained, causing hypotony. Also, the length A of the tube 11 affects the inner diameter D₁ of the tube 11 and the diameter D₂ of the ripcord 13 that may be used in the implant device 10 for an eye disease as described below.

The implant device 10 for an eye disease may have any of various shapes according to a position of a portion where the wing 12 is coupled to the tube 11. (A) of FIG. 4 illustrates an embodiment where a distance B from a distal end of the tube 11 (i.e., a portion inserted into a patient's eye chamber) to the wing 12 is about 4.4 mm, and (B) of FIG. 4 illustrates an embodiment where the distance B from the distal end of the tube 11 to the wing 12 is about 2.3 mm. In an embodiment, the distance B from the distal end of the tube 11 to the wing 12 may be about 2.3 mm to about 4.4 mm or any number or subrange therebetween.

Because the wing 12 is caught on a sclera during insertion of the implant device 10 for an eye disease to limit a range in which the tube 11 is inserted into an eyeball, as the distance B from the distal end of the tube 11 to the wing 12 increases, a portion of the tube 11 inserted into the eyeball increases. However, when the tube 11 is inserted too much into the eyeball, there may be a problem of poking the opposite side of the eyeball. Accordingly, the distance B from the distal end of the tube 11 to the wing 12 may be determined so that the wing 12 is located at an appropriate position in the tube 11 according to a skill level of an operator and the patient's condition.

A width W of the wing 12 in a direction perpendicular to a longitudinal direction A of the tube 11 may be about 0.5 mm to about 1.5 mm, or any number (e.g., about 1 mm) or subrange therebetween. Also, an overall length L of the implant device 10 for an eye disease including a length of the ripcord 13 may be about 20 mm to about 40 mm, or any number (e.g., about 30 mm) or subrange therebetween. However, these numbers are only examples, and a length, a width, a thickness, etc. of each of members constituting the implant device 10 for an eye disease may be appropriately set according to an embodiment.

### Optimization of tube length, tube inner diameter, and ripcord diameter

The implant device for an eye disease according to embodiments is inserted into an anterior chamber of an eyeball of a glaucoma patient or the like and allows aqueous humor to be drained out of an eye to lower increased intraocular pressure caused by glaucoma. In this case, the implant device for an eye disease according to embodiments is different from the prior art in that a numerical range of each of the inner diameter D₁ of the tube 11 and the diameter D₂ of the ripcord 13 is designed so that the aqueous humor is drained at an appropriate pressure.

For example, when pressure formed in the anterior chamber of the eyeball through the implant device is lower than about 6.0 mmHg, there is a problem that hypotony is caused due to excessive drainage of the aqueous humor. In contrast, when pressure formed in the anterior chamber is greater than about 20 mmHg, there is a problem that the drainage of the aqueous humor is not sufficient and intraocular pressure control fails.

To solve these problems, the applicants have completed the present disclosure by deriving a numerical range of each of (i) the length A of the tube 11, (ii) the inner diameter D₁ of the tube 11, and (iii) the diameter D₂ of the ripcord 13 in which pressure formed in the anterior chamber may be maintained within a desired range in the implant device in which the tube is inserted into the anterior chamber of the eyeball and the ripcord is inserted into a hollow portion of the tube.

In particular, the implant device for an eye disease is a device with a very special and specific purpose called an implant inserted into a specific position of the eyeball to treat an eye disease such as glaucoma, and specifications, such as detailed dimensions, of the device immediately affect the disease and health condition of the patient using the implant device. The applicants have derived a combination of dimensions of members which allows pressure formed in the anterior chamber of the eyeball to be within a desired range (e.g., about 6 mmHg to about 20 mmHg), by performing experiments and predictions through calculation while adjusting (i) the length A of the tube 11, (ii) the inner diameter D₁ of the tube 11, and (iii) the diameter D₂ of the ripcord 13 in units of micrometers (µm).

In detail, the inventors have derived an upper limit and a lower limit of a numerical range in which pressure formed in the anterior chamber may be maintained within a desired range, by setting the length A of the tube 11 to be inserted into the anterior chamber of the eyeball among elements of the implant device for an eye disease to about 4 mm to about 10 mm and changing the inner diameter D₁ of the tube 11 and the diameter D₂ of the ripcord 13 in each length of the tube.

To this end, assuming that a pressure difference before and after the tube, the amount of a fluid flowing per unit time, the inner diameter D₁ of the tube 11, the diameter D₂ of the ripcord 13, viscosity (mu) of the fluid, and the length A of the tube 11 will affect intraocular pressure according to a Navier-Stokes equation, the applicants selected numbers from among various combinations of the inner diameter D₁ of the tube 11 and the diameter D₂ of the ripcord 13 at which pressure formed in the anterior chamber may be maintained within a desired range and verified some of the numbers through experiments. In this case, it was assumed that a flow rate of the aqueous humor was about 2 µL/min and the viscosity of the aqueous humor was 0.0007191 µPa·s.

The length A of the tube 11 in the implant device 10 for an eye disease according to an embodiment of the present disclosure, derived by the above process, is about 4 mm to about 10 mm. Also, the inner diameter D₁ of the tube 11 in the implant device 10 for an eye disease according to an embodiment is about 60 µm to about 220 µm. Furthermore, the diameter D₂ of the ripcord 13 in the implant device 10 for an eye disease according to an embodiment is about 1 µm to about 180 µm.

In another embodiment, (i) the length A of the tube 11, (ii) the inner diameter D₁ of the tube 11, and (iii) the diameter D₂ of the ripcord 13 may be limited to a part of the above-described available range by considering a combination with other numerical ranges. For example, because it is difficult for a clinician to insert the ripcord 13 into the tube 11 or remove the inserted ripcord 13 when the diameter D₂ of the ripcord 13 is too small, the implant device 10 for an eye disease may be designed so that the diameter D₂ of the ripcord 13 has a range of about 20 µm to about 180 µm.

In still another embodiment, (i) the length A of the tube 11, (ii) the inner diameter D₁ of the tube 11, and (iii) the diameter D₂ of the ripcord 13 may be determined as a subrange within the above-described available range. The length A of the tube 11 in the implant device 10 for an eye disease according to an embodiment is about 5 mm to about 8 mm. Also, the inner diameter D₁ of the tube 11 in the implant device 10 for an eye disease according to an embodiment is about 60 µm to about 140 µm. Furthermore, the diameter D₂ of the ripcord 13 in the implant device 10 for an eye disease according to an embodiment is about 1 µm to about 130 µm. In still another embodiment, the diameter D₂ of the ripcord 13 is about 2 µm to about 118 µm.

Tables 1 to 4 show a perfusion pressure range obtained by the inventors through the implant device 10 by changing the inner diameter D₁ of the tube 11 and the diameter D₂ of the ripcord 13 in a state where the length A of the tube 11 is fixed to about 5 mm, about 6 mm, about 7 mm, and about 8 mm. The length and the inner diameter of the tube and the diameter of the ripcord shown in the tables of the specification are all approximate numbers.

**[Table 1]**

| | <Length of tube = 5mm> | | | | | | | | | |
|---|---|---|---|---|---|---|---|---|---|---|
| | Inner diameter of tube | | | | | | | | | |
| | | 60 | 70 | 80 | 90 | 100 | 110 | 120 | 130 | 140 |
| | 10 | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 11 | Normal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure |
| Diameter of ripcord | 25 | Normal intraocular pressure | Normal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 28 | Normal intraocular pressure | Normal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 38 | Abnormal intraocular pressure | Normal intraocular pressure | Normal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 40 | Abnormal intraocular pressure | Normal intraocular pressure | Normal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 50 | Abnormal intraocular pressure | Abnormal intraocular pressure | Normal intraocular pressure | Normal intraocular pressure | Abnormal intraocular pressure | Abnormal intraocular pressure | Abnormal intraocular pressure | Abnormal intraocular pressure | Abnormal intraocular pressure |
| | 52 | Abnormal intraocular pressure | Abnormal intraocular pressure | Normal intraocular pressure | Normal intraocular pressure | Abnormal intraocular pressure | Abnormal intraocular pressure | Abnormal intraocular pressure | Abnormal intraocular pressure | Abnormal intraocular pressure |
| | 62 | Abnormal intraocular pressure | Abnormal intraocular pressure | Abnormal intraocular pressure | Normal intraocular pressure | Normal intraocular pressure | Abnormal intraocular pressure | Abnormal intraocular pressure | Abnormal intraocular pressure | Abnormal intraocular pressure |
| | 63 | Abnormal intraocular pressure | Abnormal intraocular pressure | Abnormal intraocular pressure | Normal intraocular pressure | Normal intraocular pressure | Abnormal intraocular pressure | Abnormal intraocular pressure | Abnormal intraocular pressure | Abnormal intraocular pressure |
| | 74 | Abnormal intraocular pressure | Abnormal intraocular pressure | Abnormal intraocular pressure | Abnormal intraocular pressure | Normal intraocular pressure | Normal intraocular pressure | Abnormal intraocular pressure | Abnormal intraocular pressure | Abnormal intraocular pressure |
| | 85 | Abnormal intraocular pressure | Abnormal intraocular pressure | Abnormal intraocular pressure | Abnormal intraocular pressure | Abnormal intraocular pressure | Normal intraocular pressure | Normal intraocular pressure | Abnormal intraocular pressure | Abnormal intraocular pressure |
| | 96 | Abnormal intraocular pressure | Abnormal intraocular pressure | Abnormal intraocular pressure | Abnormal intraocular pressure | Abnormal intraocular pressure | Abnormal intraocular pressure | Normal intraocular pressure | Normal intraocular pressure | Abnormal intraocular pressure |
| | 107 | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Normal intraocular pressure | Normal intraocular pressure |
| | 118 | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Normal intraocular pressure |
| | 119 | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure |

**[Table 2]**

| | **<Length of tube = 6mm>** | | | | | | | | | |
|---|---|---|---|---|---|---|---|---|---|---|
| | **Inner diameter of tube** | | | | | | | | | |
| | | 60 | 70 | 80 | 90 | 100 | 110 | 120 | 130 | 140 |
| | 6 | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure |
| **Diameter of ripcord** | 7 | Normal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 21 | Normal intraocular pressure | Normal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 26 | Normal intraocular pressure | Normal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 35 | Abnormal intraocular pressure | Normal intraocular pressure | Normal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 38 | Abnormal intraocular pressure | Normal intraocular pressure | Normal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 47 | Abnormal intraocular pressure | Abnormal intraocular pressure | Normal intraocular pressure | Normal intraocular pressure | Abnormal intraocular pressure | Abnormal intraocular pressure | Abnormal intraocular pressure | Abnormal intraocular pressure | Abnormal intraocular pressure |
| | 50 | Abnormal intraocular pressure | Abnormal intraocular pressure | Normal intraocular pressure | Normal intraocular pressure | Abnormal intraocular pressure | Abnormal intraocular pressure | Abnormal intraocular pressure | Abnormal intraocular pressure | Abnormal intraocular pressure |
| | 60 | Abnormal intraocular pressure | Abnormal intraocular pressure | Abnormal intraocular pressure | Normal intraocular pressure | Normal intraocular pressure | Abnormal intraocular pressure | Abnormal intraocular pressure | Abnormal intraocular pressure | Abnormal intraocular pressure |
| | 62 | Abnormal intraocular pressure | Abnormal intraocular pressure | Abnormal intraocular pressure | Normal intraocular pressure | Normal intraocular pressure | Abnormal intraocular pressure | Abnormal intraocular pressure | Abnormal intraocular pressure | Abnormal intraocular pressure |
| | 71 | Abnormal intraocular pressure | Abnormal intraocular pressure | Abnormal intraocular pressure | Abnormal intraocular pressure | Normal intraocular pressure | Normal intraocular pressure | Abnormal intraocular pressure | Abnormal intraocular pressure | Abnormal intraocular pressure |
| | 73 | Abnormal intraocular pressure | Abnormal intraocular pressure | Abnormal intraocular pressure | Abnormal intraocular pressure | Normal intraocular pressure | Normal intraocular pressure | Abnormal intraocular pressure | Abnormal intraocular pressure | Abnormal intraocular pressure |
| | 83 | Abnormal intraocular pressure | Abnormal intraocular pressure | Abnormal intraocular pressure | Abnormal intraocular pressure | Abnormal intraocular pressure | Normal intraocular pressure | Normal intraocular pressure | Abnormal intraocular pressure | Abnormal intraocular pressure |
| | 84 | Abnormal intraocular pressure | Abnormal intraocular pressure | Abnormal intraocular pressure | Abnormal intraocular pressure | Abnormal intraocular pressure | Normal intraocular pressure | Normal intraocular pressure | Abnormal intraocular pressure | Abnormal intraocular pressure |
| | 94 | Abnormal intraocular pressure | Abnormal intraocular pressure | Abnormal intraocular pressure | Abnormal intraocular pressure | Abnormal intraocular pressure | Abnormal intraocular pressure | Normal intraocular pressure | Normal intraocular pressure | Abnormal intraocular pressure |
| | 95 | Abnormal intraocular pressure | Abnormal intraocular pressure | Abnormal intraocular pressure | Abnormal intraocular pressure | Abnormal intraocular pressure | Abnormal intraocular pressure | Normal intraocular pressure | Normal intraocular pressure | Abnormal intraocular pressure |
| | 105 | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Normal intraocular pressure | Normal intraocular pressure |
| | 116 | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Normal intraocular pressure |
| | 117 | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure |

**[Table 3]**

| | **<Length of tube = 7mm>** | | | | | | | | | |
|---|---|---|---|---|---|---|---|---|---|---|
| | **Inner diameter of tube** | | | | | | | | | |
| | | 60 | 70 | 80 | 90 | 100 | 110 | 120 | 130 | 140 |
| | 3 | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 4 | Normal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure |
| **Diameter of ripcord** | 18 | Normal intraocular pressure | Normal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 24 | Normal intraocular pressure | Normal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 32 | Abnormal intraocular pressure | Normal intraocular pressure | Normal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 37 | Abnormal intraocular pressure | Normal intraocular pressure | Normal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 45 | Abnormal intraocular pressure | Abnormal intraocular pressure | Normal intraocular pressure | Normal intraocular pressure | Abnormal intraocular pressure | Abnormal intraocular pressure | Abnormal intraocular pressure | Abnormal intraocular pressure | Abnormal intraocular pressure |
| | 49 | Abnormal intraocular pressure | Abnormal intraocular pressure | Normal intraocular pressure | Normal intraocular pressure | Abnormal intraocular pressure | Abnormal intraocular pressure | Abnormal intraocular pressure | Abnormal intraocular pressure | Abnormal intraocular pressure |
| | 57 | Abnormal intraocular pressure | Abnormal intraocular pressure | Abnormal intraocular pressure | Normal intraocular pressure | Normal intraocular pressure | Abnormal intraocular pressure | Abnormal intraocular pressure | Abnormal intraocular pressure | Abnormal intraocular pressure |
| | 60 | Abnormal intraocular pressure | Abnormal intraocular pressure | Abnormal intraocular pressure | Normal intraocular pressure | Normal intraocular pressure | Abnormal intraocular pressure | Abnormal intraocular pressure | Abnormal intraocular pressure | Abnormal intraocular pressure |
| | 69 | Abnormal intraocular pressure | Abnormal intraocular pressure | Abnormal intraocular pressure | Abnormal intraocular pressure | Normal intraocular pressure | Normal intraocular pressure | Abnormal intraocular pressure | Abnormal intraocular pressure | Abnormal intraocular pressure |
| | 71 | Abnormal intraocular pressure | Abnormal intraocular pressure | Abnormal intraocular pressure | Abnormal intraocular pressure | Normal intraocular pressure | Normal intraocular pressure | Abnormal intraocular pressure | Abnormal intraocular pressure | Abnormal intraocular pressure |
| | 81 | Abnormal intraocular pressure | Abnormal intraocular pressure | Abnormal intraocular pressure | Abnormal intraocular pressure | Abnormal intraocular pressure | Normal intraocular pressure | Normal intraocular pressure | Abnormal intraocular pressure | Abnormal intraocular pressure |
| | 82 | Abnormal intraocular pressure | Abnormal intraocular pressure | Abnormal intraocular pressure | Abnormal intraocular pressure | Abnormal intraocular pressure | Normal intraocular pressure | Normal intraocular pressure | Abnormal intraocular pressure | Abnormal intraocular pressure |
| | 92 | Abnormal intraocular pressure | Abnormal intraocular pressure | Abnormal intraocular pressure | Abnormal intraocular pressure | Abnormal intraocular pressure | Abnormal intraocular pressure | Normal intraocular pressure | Normal intraocular pressure | Abnormal intraocular pressure |
| | 93 | Abnormal intraocular pressure | Abnormal intraocular pressure | Abnormal intraocular pressure | Abnormal intraocular pressure | Abnormal intraocular pressure | Abnormal intraocular pressure | Normal intraocular pressure | Normal intraocular pressure | Abnormal intraocular pressure |
| | 103 | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Normal intraocular pressure | Normal intraocular pressure |
| | 104 | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Normal intraocular pressure | Normal intraocular pressure |
| | 115 | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Normal intraocular pressure |
| | 116 | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure |

**[Table 4]**

| | **<Length of tube = 8mm>** | | | | | | | | | |
|---|---|---|---|---|---|---|---|---|---|---|
| | **Inner diameter of tube** | | | | | | | | | |
| **Diameter of ripcord** | | 60 | 70 | 80 | 90 | 100 | 110 | 120 | 130 | 140 |
| | 1 | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 2 | Normal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 16 | Normal intraocular pressure | Normal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 22 | Normal intraocular pressure | Normal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 30 | Abnormal intraocular pressure | Normal intraocular pressure | Normal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 35 | Abnormal intraocular pressure | Normal intraocular pressure | Normal intraocular pressure | Abnormal intraocular pressure | Abnormal intraocular pressure | Abnormal intraocular pressure | Abnormal intraocular pressure | Abnormal intraocular pressure | Abnormal intraocular pressure |
| | 43 | Abnormal intraocular pressure | Abnormal intraocular pressure | Normal intraocular pressure | Normal intraocular pressure | Abnormal intraocular pressure | Abnormal intraocular pressure | Abnormal intraocular pressure | Abnormal intraocular pressure | Abnormal intraocular pressure |
| | 47 | Abnormal intraocular pressure | Abnormal intraocular pressure | Normal intraocular pressure | Normal intraocular pressure | Abnormal intraocular pressure | Abnormal intraocular pressure | Abnormal intraocular pressure | Abnormal intraocular pressure | Abnormal intraocular pressure |
| | 55 | Abnormal intraocular pressure | Abnormal intraocular pressure | Abnormal intraocular pressure | Normal intraocular pressure | Normal intraocular pressure | Abnormal intraocular pressure | Abnormal intraocular pressure | Abnormal intraocular pressure | Abnormal intraocular pressure |
| | 59 | Abnormal intraocular pressure | Abnormal intraocular pressure | Abnormal intraocular pressure | Normal intraocular pressure | Normal intraocular pressure | Abnormal intraocular pressure | Abnormal intraocular pressure | Abnormal intraocular pressure | Abnormal intraocular pressure |
| | 67 | Abnormal intraocular pressure | Abnormal intraocular pressure | Abnormal intraocular pressure | Abnormal intraocular pressure | Normal intraocular pressure | Normal intraocular pressure | Abnormal intraocular pressure | Abnormal intraocular pressure | Abnormal intraocular pressure |
| | 70 | Abnormal intraocular pressure | Abnormal intraocular pressure | Abnormal intraocular pressure | Abnormal intraocular pressure | Normal intraocular pressure | Normal intraocular pressure | Abnormal intraocular pressure | Abnormal intraocular pressure | Abnormal intraocular pressure |
| | 79 | Abnormal intraocular pressure | Abnormal intraocular pressure | Abnormal intraocular pressure | Abnormal intraocular pressure | Abnormal intraocular pressure | Normal intraocular pressure | Normal intraocular pressure | Abnormal intraocular pressure | Abnormal intraocular pressure |
| | 81 | Abnormal intraocular pressure | Abnormal intraocular pressure | Abnormal intraocular pressure | Abnormal intraocular pressure | Abnormal intraocular pressure | Normal intraocular pressure | Normal intraocular pressure | Abnormal intraocular pressure | Abnormal intraocular pressure |
| | 90 | Abnormal intraocular pressure | Abnormal intraocular pressure | Abnormal intraocular pressure | Abnormal intraocular pressure | Abnormal intraocular pressure | Abnormal intraocular pressure | Normal intraocular pressure | Normal intraocular pressure | Abnormal intraocular pressure |
| | 92 | Abnormal intraocular pressure | Abnormal intraocular pressure | Abnormal intraocular pressure | Abnormal intraocular pressure | Abnormal intraocular pressure | Abnormal intraocular pressure | Normal intraocular pressure | Normal intraocular pressure | Abnormal intraocular pressure |
| | 101 | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Normal intraocular pressure | Normal intraocular pressure |
| | 103 | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Normal intraocular pressure | Normal intraocular pressure |
| | 114 | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Normal intraocular pressure |
| | 115 | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure | Abnormal intraocular pressure |

Also, Table 5 shows a combination of numbers at which normal intraocular pressure may be obtained from results shown in Tables 1 to 4.

**[Table 5]**

| Length A of the tube 11 | Inner diameter D₁ of the tube 11 | Diameter D₂ of the ripcord 13 |
|---|---|---|
| 5 mm | 60 to 140 µm | 11 to 118 µm |
| 6 mm | 60 to 140 µm | 7 to 116 µm |
| 7 mm | 60 to 140 µm | 4 to 115 µm |
| 8 mm | 60 to 140 µm | 2 to 114 µm |

As shown in Table 5, in the implant device 10 for an eye disease according to an embodiment, under the condition that the length A of the tube 11 is about 5 mm to about 8 mm, the inner diameter D₁ of the tube 11 may be about 60 µm to about 140 µm. Also, in the implant device 10 for an eye disease according to an embodiment, the diameter D₂ of the ripcord 13 may be about 1 µm to about 130 µm. In still another embodiment, the diameter D₂ of the ripcord 13 may be about 11 µm to about 130 µm. In still another embodiment, the diameter D₂ of the ripcord 13 may be about 20 µm to about 130 µm. In still another embodiment, the diameter D₂ of the ripcord 13 may be about 20 µm to about 118 µm.

Also, the inventors investigated combinations of numbers for obtaining normal intraocular pressure by increasing the inner diameter D₁ of the tube 11 by 10 µm in a range of about 60 µm to about 200 µm and changing the length A of the tube 11 and the diameter D₂ of the ripcord 13 under the given condition of the inner diameter D₁ of the tube 11, and a result is shown in Table 6.

**[Table 6]**

| Inner diameter D₁ of the tube 11 | Length A of the tube 11 | Diameter D₂ of the ripcord 13 |
|---|---|---|
| 60 µm | 4 to 9 mm | 20 to 30 µm |
| 70 µm | 4 to 10 mm | 20 to 40 µm |
| 80 µm | 4 to 10 mm | 35 to 45 µm |
| 90 µm | 4 to 10 mm | 40 to 65 µm |
| 100 µm | 4 to 10 mm | 55 to 75 µm |
| 110 µm | 4 to 10 mm | 65 to 85 µm |
| 120 µm | 4 to 10 mm | 80 to 95 µm |
| 130 µm | 4 to 10 mm | 95 to 105 µm |
| 140 µm | 4 to 10 mm | 100 to 115 µm |
| 150 µm | 4 to 10 mm | 110 to 130 µm |
| 160 µm | 4 to 10 mm | 120 to 140 µm |
| 170 µm | 4 to 10 mm | 135 to 145 µm |
| 180 µm | 4 to 10 mm | 145 to 160 µm |
| 190 µm | 4 to 10 mm | 155 to 170 µm |
| 200 µm | 4 to 10 mm | 165 to 180 µm |

As shown in Table 6, in the implant device 10 for an eye disease according to an embodiment, under the condition that the inner diameter D₁ of the tube 11 is about 60 µm to about 200 µm, the length A of the tube 11 may be about 4 mm to about 10 mm, or may be determined in a range of about 4 mm to about 9 mm, which is smaller than the range of about 4 mm to about 10 mm. Also, in the implant device 10 for an eye disease according to an embodiment, the diameter D₂ of the ripcord 13 may be about 20 µm to about 180 µm.

As shown in a result in the above table, the inner diameter D₁ of the tube 11 and the diameter D₂ of the ripcord 13 may be determined to have a preset ratio therebetween in a combination of numbers for obtaining normal intraocular pressure.

For example, the implant device 10 for an eye disease according to an embodiment may be designed so that the inner diameter D₁ of the tube 11 and the diameter D₂ of the ripcord 13 have a certain ratio, or a square D₁² of the inner diameter of the tube 11 and a square D₂² of the diameter of the ripcord 13 have a certain ratio. For example, it is assumed that a ratio between the inner diameter D₁ of the tube 11 and the diameter D₂ of the ripcord 13, or a ratio between the square of the inner diameter of the tube 11 and the square of the diameter of the ripcord 13 is expressed as abut 1:a to about 1:b. When the length A of the tube was about 5 mm and the inner diameter D₁ of the tube 11 was about 60 µm, the diameter D₂ of the ripcord 13 at which normal intraocular pressure was obtained was about 11 µm to about 28 µm, and in this case, a range of a ratio between the inner diameter D₁ of the tube 11 and the diameter D₂ of the ripcord 13 may be about 1:0.18 to about 1:0.47 or any number or subrange therebetween.

In an embodiment, the implant device for an eye disease may be designed so that a and b respectively defining a lower limit and an upper limit of a ratio between the inner diameter D₁ of the tube 11 and the diameter D₂ of the ripcord 13 increase as the inner diameter D₁ of the tube 11 increases. That is, when the inner diameter D₁ of the tube 11 is small, normal intraocular pressure may be obtained only by using a ripcord of the diameter D₂ having a ratio of about 3:1 to about 2:1 to the inner diameter D₁ of the tube 11, but as the inner diameter D₁ of the tube 11 increases, normal intraocular pressure may be obtained even when a ripcord of a larger diameter D₂ having a ratio of about 2:1 to about 1.75:1 to the inner diameter D₁ of the tube 11 is used.

Table 7 shows a ratio between the inner diameter D₁ of the tube 11 and the diameter D₂ of the ripcord 13 organized by the inventors, based on a ratio between the inner diameter D₁ of the tube 11 and the diameter D₂ of the ripcord 13 at which normal intraocular pressure may be obtained when the length A of the tube 11 is about 5 mm, about 6 mm, about 7 mm, and about 8 mm.

**[Table 7]**

| Inner diameter D₁ of the tube | Diameter D₂ of the ripcord | | | | Ratio of inner diameter D₁ of the tube : diameter D₂ of the ripcord |
|---|---|---|---|---|---|
| | Length of the tube (5mm) | Length of the tube (6mm) | Length of the tube (7mm) | Length of the tube (8mm) | |
| 60 | 1:0.18 to 1:0.47 | 1:0.12 to 1:0.43 | 1:0.07 to 1:0.40 | 1:0.03 to 1:0.37 | 1:0.01 to 1:0.5 |
| 70 | 1:0.36 to 1:0.57 | 1:0.30 to 1:0.54 | 1:0.26 to 1:0.53 | 1:0.23 to 1:0.50 | 1:0.2 to 1:0.6 |
| 80 | 1:0.48 to 1:0.65 | 1:0.44 to 1:0.63 | 1:0.40 to 1:0.61 | 1:0.38 to 1:0.59 | 1:0.3 to 1:0.7 |
| 90 | 1:0.56 to 1:0.70 | 1:0.52 to 1:0.69 | 1:0.50 to 1:0.67 | 1:0.48 to 1:0.66 | 1:0.4 to 1:0.8 |
| 100 | 1:0.62 to 1:0.74 | 1:0.60 to 1:0.73 | 1:0.57 to 1:0.71 | 1:0.55 to 1:0.70 | 1:0.5 to 1:0.8 |
| 110 | 1:0.67 to 1:0.77 | 1:0.65 to 1:0.76 | 1:0.63 to 1:0.75 | 1:0.61 to 1:0.74 | 1:0.6 to 1:0.8 |
| 120 | 1:0.71 to 1:0.80 | 1:0.69 to 1:0.79 | 1:0.68 to 1:0.78 | 1:0.66 to 1:0.77 | 1:0.6 to 1:0.9 |
| 130 | 1:0.74 to 1:0.82 | 1:0.72 to 1:0.81 | 1:0.71 to 1:0.80 | 1:0.69 to 1:0.79 | 1:0.6 to 1:0.9 |
| 140 | 1:0.76 to 1:0.84 | 1:0.75 to 1:0.83 | 1:0.74 to 1:0.82 | 1:0.72 to 1:0.81 | 1:0.7 to 1:0.9 |

In an embodiment, a ratio between the inner diameter D₁ of the tube 11 and the diameter D₂ of the ripcord 13 may be about 1:0.01 to about 1:0.9 or any number or subrange therebetween. Also, in an embodiment, a ratio between the inner diameter D₁ of the tube 11 and the diameter D₂ of the ripcord 13 may be determined so that a lower limit and/or an upper limit of the ratio increases as the inner diameter D₁ of the tube 11 increases, as shown in Table 7.

In an embodiment, when the inner diameter D₁ of the tube 11 is about 60 µm, a ratio between the inner diameter D₁ of the tube 11 and the diameter D₂ of the ripcord 13 may be about 1:0.03 to about 1:0.47 or any number or subrange therebetween.

In an embodiment, when the inner diameter D₁ of the tube 11 is about 70 µm, a ratio between the inner diameter D₁ of the tube 11 and the diameter D₂ of the ripcord 13 may be about 1:0.23 to about 1:0.57 or any number or subrange therebetween.

In an embodiment, when the inner diameter D₁ of the tube 11 is about 80 µm, a ratio between the inner diameter D₁ of the tube 11 and the diameter D₂ of the ripcord 13 may be about 1:0.38 to about 1:0.65 or any number or subrange therebetween.

In an embodiment, when the inner diameter D₁ of the tube 11 is about 90 µm, a ratio between the inner diameter D₁ of the tube 11 and the diameter D₂ of the ripcord 13 may be about 1:0.48 to about 1:0.70 or any number or subrange therebetween.

In an embodiment, when the inner diameter D₁ of the tube 11 is about 100 µm, a ratio between the inner diameter D₁ of the tube 11 and the diameter D₂ of the ripcord 13 may be about 1:0.55 to about 1:0.74 or any number or subrange therebetween.

In an embodiment, when the inner diameter D₁ of the tube 11 is about 110 µm, a ratio between the inner diameter D₁ of the tube 11 and the diameter D₂ of the ripcord 13 may be about 1:0.61 to about 1:0.77 or any number or subrange therebetween.

In an embodiment, when the inner diameter D₁ of the tube 11 is about 120 µm, a ratio between the inner diameter D₁ of the tube 11 and the diameter D₂ of the ripcord 13 may be about 1:0.66 to about 1:0.80 or any number or subrange therebetween.

In an embodiment, when the inner diameter D₁ of the tube 11 is about 130 µm, a ratio between the inner diameter D₁ of the tube 11 and the diameter D₂ of the ripcord 13 may be about 1:0.69 to about 1:0.82 or any number or subrange therebetween.

In an embodiment, when the inner diameter D₁ of the tube 11 is about 140 µm, a ratio between the inner diameter D₁ of the tube 11 and the diameter D₂ of the ripcord 13 may be about 1:0.72 to about 1:0.84 or any number or subrange therebetween.

Also, in the implant device for an eye disease according to an embodiment, as the inner diameter D₁ of the tube 11 increases, the diameter D₂ of the ripcord 13 that may be used may increase at a certain ratio with respect to an increment in the inner diameter D₁ of the tube 11. Table 8 shows a lower limit and an upper limit of the diameter D₂ of the ripcord 13 designed by the inventors to obtain normal intraocular pressure by sequentially increasing the inner diameter D₁ of the tube 11 from 60 µm by 10 µm when the length A of the tube 11 is about 5 mm, about 6 mm, about 7 mm, and about 8 mm.

**[Table 8]**

| Inner diameter D₁ of the tube | Increase in the diameter D₂ of the ripcord | | | | | | | |
|---|---|---|---|---|---|---|---|---|
| | Length of the tube (5mm) | | Length of the tube (6mm) | | Length of the tube (7mm) | | Length of the tube (8mm) | |
| | Lower limit | Upper limit | Lower limit | Upper limit | Lower limit | Upper limit | Lower limit | Upper limit |
| 70 | 14 | 12 | 14 | 12 | 14 | 13 | 14 | 13 |
| 80 | 13 | 12 | 14 | 12 | 14 | 12 | 14 | 12 |
| 90 | 12 | 11 | 12 | 12 | 13 | 11 | 13 | 12 |
| 100 | 12 | 11 | 13 | 11 | 12 | 11 | 12 | 11 |
| 110 | 12 | 11 | 11 | 11 | 12 | 11 | 12 | 11 |
| 120 | 11 | 11 | 12 | 11 | 12 | 11 | 12 | 11 |
| 130 | 11 | 11 | 11 | 10 | 11 | 11 | 11 | 11 |
| 140 | 11 | 11 | 11 | 11 | 11 | 11 | 11 | 11 |

As shown in Table 8, in the implant device for an eye disease according to an embodiment, the length A of the tube 11 is about 5 mm to about 8 mm, and the inner diameter D₁ of the tube 11 is about 60 µm to about 140 µm. In this case, regarding a lower limit and an upper limit of the diameter D₂ of the ripcord 13 that may be used, a ratio between the inner diameter D₁ of the tube 11 and the diameter D₂ of the ripcord 13 may be designed so that the lower limit and/or the upper limit of the diameter D₂ of the ripcord 13 increases by about 1 to about 1.5 times an increment in the inner diameter D₁ of the tube 11 whenever the inner diameter D₁ of the tube 11 increases.

In another embodiment, a ratio between the inner diameter D₁ of the tube 11 and the diameter D₂ of the ripcord 13 may be designed so that the lower limit and/or the upper limit of the diameter D₂ of the ripcord 13 increases by any number or subrange within a range of about 10 µm to about 15 µm whenever the inner diameter D₁ of the tube 11 increases by about 10 µm. For example, a ratio between the inner diameter D₁ of the tube 11 and the diameter D₂ of the ripcord 13 may be designed so that the lower limit and/or the upper limit of the diameter D₂ of the ripcord 13 increases by about 10 µm to about 14 µm whenever the inner diameter D₁ of the tube 11 increases by about 10 µm.

Also, in an embodiment, a ratio between the inner diameter D₁ of the tube 11 and the diameter D₂ of the ripcord 13 may be designed so that as the inner diameter D₁ of the tube 11 increases, an increase in the lower limit and the upper limit of the diameter D₂ of the ripcord 13 decreases, and ultimately converges to a specific value (e.g., about 10 µm to about 12 µm).

Although the inner diameter D₁ of the tube 11 and the diameter D₂ of the ripcord 13 have a certain ratio for convenience of explanation, the implant device 10 for an eye disease may be designed by applying the same principle to a ratio between the square D₁² of the inner diameter of the tube 11 and the square D₂² of the diameter of the ripcord 13.

Also, in an embodiment, the implant device 10 for an eye disease according to an embodiment may be designed so that under the condition that the inner diameter D₁ of the tube 11 and the diameter D₂ of the ripcord 13 satisfy the above ratio, as the length A of the tube 11 becomes longer, the ripcord 13 having a smaller diameter D₂ is used (i.e., a lower limit and/or an upper limit of a ratio of the diameter D₂ of the ripcord 13 to the inner diameter D₁ of the tube 11 decreases). This is because the longer the length A of the tube 11, the greater the resistance received by the aqueous humor to pass through the implant device 10, and thus, it is necessary to secure a wider space between an inner wall of the tube 11 and the ripcord 13.

The above description of the present disclosure is provided for illustration, and it will be understood by one of ordinary skill in the art that various changes in form and details may be readily made therein without departing from essential features and the scope of the present disclosure. Accordingly, the above embodiments are examples only in all aspects and are not limited. For example, each component described as a single type may be executed in a distributed manner, and components described as a distributed type may be executed in a combined manner.

The scope of the present disclosure is indicated by the claims rather than by the detailed description of the present disclosure, and it should be understood that the claims and all modifications or modified forms drawn from the concept and scope of the claims and equivalents are included in the scope of the present disclosure.

## Claims

1. An implant device for an eye disease to be inserted into an eyeball, the implant device comprising:
a tube having one end to be inserted into an anterior chamber of the eyeball, the tube comprising a hollow portion through which aqueous humor is drained; and
a ripcord to be inserted into the hollow portion of the tube to control an amount of aqueous humor drainage through the tube,
wherein an inner diameter of the tube and a diameter of the ripcord are determined so that pressure formed in the anterior chamber of the eyeball when the tube is inserted into the anterior chamber of the eyeball is within a preset pressure range.

2. The implant device for an eye disease according to claim 1, wherein the preset pressure range is about 6 mmHg to about 20 mmHg.

3. The implant device for an eye disease according to claim 2, wherein
a length of the tube is about 4 mm to about 10 mm,
the inner diameter of the tube is about 60 µm to about 220 µm, and
the diameter of the ripcord is about 1 µm to about 180 µm.

4. The implant device for an eye disease according to claim 2, wherein
a length of the tube is about 5 mm to about 8 mm,
the inner diameter of the tube is about 60 µm to about 140 µm, and
the diameter of the ripcord is about 1 µm to about 130 µm.

5. The implant device for an eye disease according to claim 1, wherein at least one of the inner diameter of the tube and the diameter of the ripcord is determined so that a ratio of the diameter of the ripcord to the inner diameter of the tube is between a preset lower limit and a preset upper limit.

6. The implant device for an eye disease according to claim 5, wherein the ratio of the diameter of the ripcord to the inner diameter of the tube is about 1:0.01 to about 1:0.9.

7. The implant device for an eye disease according to claim 5, wherein at least one of the preset upper limit and the preset lower limit is determined to increase as the inner diameter of the tube increases.

8. The implant device for an eye disease according to claim 7, wherein an increment in the at least one of the preset upper limit and the preset lower limit, as the inner diameter of the tube increases, decreases as the inner diameter of the tube increases.

9. The implant device for an eye disease according to claim 5, wherein at least one of the preset upper limit and the preset lower limit is determined to decrease as a length of the tube increases.

10. The implant device for an eye disease according to claim 1, wherein at least one of the inner diameter of the tube and the diameter of the ripcord is determined so that a ratio of a square of the diameter of the ripcord to a square of the inner diameter of the tube is between a preset lower limit and a preset upper limit.

11. The implant device for an eye disease according to claim 1, further comprising a wing coupled to the tube to be located at a preset distance from a distal end of the tube and extending in a direction different from a longitudinal direction of the tube.

12. A method for maintaining a predetermined intraocular pressure of a subject in need thereof, comprising inserting an implant device for an eye disease according to any of the preceding claims into an anterior chamber of an eyeball of the subject.
